# EUROPEAN PATENT APPLICATION

(11) **EP 2 098 222 A1**
(43) Date of publication of application: **09.09.2009**
(21) Application number: 07830958.0
(22) Date of filing: 31.10.2007
(51) Int. Cl.: A61K 9/14, A61K 9/10, A61K 9/20, A61K 31/07, A61K 31/122, A61K 31/203, A61K 31/355, A61K 31/59, A61K 36/48, A61K 47/02, A61K 47/14, A61K 47/22, A61K 47/24, A61K 47/26, A61K 47/32, A61K 47/36, A61K 47/38, A61K 47/42, A61P 3/02, A61P 9/04, A61P 39/06

(54) **PHYSIOLOGICALLY ACTIVE SUBSTANCE-CONTAINING GRANULAR COMPOSITION AND METHOD OF PRODUCING THE SAME**

(30) Priority: 31.10.2006 JP 2006295426; 07.11.2006 US 857111 P
(71) Applicant: Kaneka Corporation, Kita-ku Osaka-shi, Osaka 530-8288 (JP)
(72) Inventor: NAGIRA, Yozo, 662-0857 NISHINOMIYA-SHI HYOGO (JP); IKEHARA, Toshinori, Takasago-shi, Hyogo 676-0011 (JP); UEDA, Takashi, Kakogawa-shi, Hyogo 675-0301 (JP); AKAO, Shinsuke, Takasago-shi, Hyogo 676-0078 (JP)
(74) Representative: Hart-Davis, Jason
(86) International application number: PCT/JP2007/071224
(87) International publication number: WO 2008/053920

(57) **Abstract**

The present invention provides a particulate composition wherein an oil component (A) comprising a poorly water-soluble bioactive substance is poly-dispersed while forming a domain in a matrix comprising of a water-soluble excipient comprising a water-soluble polymer as a main component, and wherein the sphericity of the particulate composition is not less than 0.9 and a method of producing the same. The particulate composition simultaneously has excellent water solubility, workability, tabletability and heat-resistance. It also provides a food, food with nutrient function claims, food for specified health uses, nutritional supplement, nutritional product, animal drug, drink, feed, pet food, cosmetic, pharmaceutical product, therapeutic drug, prophylactic drug and the like, which contain the particulate composition.

## Description

### Technical Field

The present invention relates to a particulate composition comprising a bioactive substance and a method of producing the composition. More specifically, the present invention relates to a particulate composition comprising a bioactive substance, having both powder characteristics allowing easy handling and high oral absorbability, and a method of producing the composition.

### Background Art

For poorly water-soluble or oil-soluble bioactive substances, their processing into tablets and their addition directly to foods such as beverages are difficult, and their absorbability in oral ingestion is low in some cases. For example, coenzyme Q10 is an oil-soluble crystalline powder having a melting point of about 48°C, and is known to have a very low absorbability in oral ingestion because it is hardly soluble in water.

Coenzyme Q₁₀ is a physiological component present as a constituent component of the mitochondrial electron transport system in the cell of the living body. It functions as a transport component in the electron transport system by repeating oxidation and reduction in the living body. Coenzyme Q₁₀ is known to show energy production, membrane stabilization and antioxidant activity in the living body, and has a high degree of usability. Coenzyme Q₁₀ occurs in two forms, the oxidized form and the reduced form, and it is known that, in the living body, usually about 40 to 90% of the coenzyme exists in the reduced form. Of coenzymes Q_{10,}oxidized coenzyme Q₁₀ (aka. ubiquinone or ubidecarenone) is widely used for pharmaceutical field as a drug for congestive heart failure. Besides the pharmaceutical use, it is widely used as an agent for oral preparation and a skin preparation, or as a nutritional product or a nutritional supplement, like vitamin. More recently, oxidized coenzyme Q10 has been approved for use as a food in Japan, and it is drawing attention as a constituent for health foods. It is known, however, that oxidized coenzyme Q10 poses problems in poor powder fluidity and tableting damage when used as is for hard capsules and tableting.

To solve these problems, a wide variety of technical proposals have been made to date. For example, as the most common method of producing a powder preparation comprising oxidized coenzyme Q10, spray drying in a gas phase (spray drying method) can be mentioned (patent references 1 and 2). Although this process is advantageous in that the number of steps is relatively small, a major problem exists with the low recovery rate. Because the fluidity of the powder obtained by the spray drying method is poor, or because static electricity is likely to occur in some cases, or for other reasons, the powder is difficult to use as is and requires an additional granulation step.

Meanwhile, the spray cooler method (patent reference 3), a method of producing oxidized coenzyme Q10 powder preparations, is advantageous over the spray drying method because of its better recoverability, but is problematic in operational complexity in the granulation step and the drying step at ultra-low temperature, limited excipient (gelatin only), and poor heat-resistance of the preparations and others. In addition, the spray cooler method and the spray drying method require specially designed expensive equipment, and are not considered reasonable in terms of production costs.

Although solubilized powders of oxidized coenzyme Q10 and methods of preparing the same have been developed to date, many of them have only limited uses, such as for either tablets or foods. In this situation, there has been a demand for oxidized coenzyme Q10 powder preparations of high contents of oxidized coenzyme Q10 that can be utilized not only for tablets and hard capsules, but also for all kinds of foods, including beverages, confectionery, and milk products, and that have excellent water solubility, workability and heat-resistance.

For the same reason as with the above-described coenzyme Q10, there has been a demand for powder preparations having excellent water solubility and workability from poorly water-soluble bioactive substances, for example, unsaturated fatty acids, oil-soluble vitamins, plant hydrophobic extract ingredients and carotenoids, and the like.
patent reference 1: JP-A-2006-022187
patent reference 2: JP-A-2003-300870
patent reference 3: JP-A-2006-089381

### Disclosure of the Invention

### Problems to Be Solved by the Invention

To solve the above-described problems, the present invention is directed to propose a particulate composition comprising a bioactive substance and a method of producing the composition to be used in the fields of foods, foods with nutrient function claims, foods for specified health uses, nutritional supplements, nutritional products, animal drugs, beverages, feeds, pet foods, cosmetics, pharmaceuticals, therapeutic drugs, prophylactic drugs and the like.

### Means for Solving the Problems

The present inventors diligently investigated to solve the above-described problems and, as a result, found that a particulate composition of excellent sphericity wherein an oil component comprising a poorly water-soluble bioactive substance is poly-dispersed while forming a domain in a matrix comprising a water-soluble excipient comprising a water-soluble polymer as a main component, is a composition excellent in all of water solubility, workability, tabletability and heat-resistance, established a method for producing the particulate composition, and developed the present invention.

Thus, the present invention provides the following.
[1] A particulate composition wherein an oil component (A) comprising a poorly water-soluble bioactive substance is poly-dispersed while forming a domain in a matrix comprising a water-soluble excipient comprising a water-soluble polymer as a main component, and wherein the sphericity is not less than 0.9.
[2] The particulate composition of [1], wherein the poorly water-soluble bioactive substance is at least one kind selected from the group consisting of oxidized coenzyme Q10, an unsaturated fatty acid, an oil-soluble vitamin, a plant hydrophobic extract ingredient and a carotenoid.
[3] The particulate composition of [1] or [2], wherein the oil component (A) is poly-dispersed while forming not less than five domains.
[4] The particulate composition of any one of [1] to [3], wherein the water-soluble polymer is at least one kind selected from the group consisting of gum arabic, ghatti gum, agar, starch, pectin, carrageenan, casein, dried albumen, curdlan, alginic acids, soybean polysaccharide, pullulan, celluloses, xanthan gum, carmellose salt and polyvinylpyrrolidone.
[5] The particulate composition of [2], wherein the unsaturated fatty acid is at least one kind selected from the group consisting of oleic acid, linoleic acid, conjugated linoleic acid, linolenic acid, arachidonic acid, docosahexaenoic acid, docosapentaenoic acid and eicosapentaenoic acid.
[6] The particulate composition of [2], wherein the oil-soluble vitamin is at least one kind selected from the group consisting of vitamin A, vitamin D, vitamin E, vitamin K, and derivatives thereof.
[7] The particulate composition of [2], wherein the plant hydrophobic extract ingredient is obtained from at least one kind of plants or plant-processed products selected from the group consisting of licorice, turmeric, perilla, clove, cinnamon, ginger, lemongrass, peppermint, dokudami (Houttuyania cordata), coix seed, rice bran, cornflower, fennel, boxthorn, zanthoxylum, nasturtium, dioscorea, sanryo, kinkaran (Tinospora capillipes), amachazuru (Gynostema pentaphyllum), thuja, hakutouou (Pulsatilla chinensis), parsley, onion, nutmeg, wild-rice, gluten feed, tobiko (konnyaku byproduct powder), paprika, horseradish, lemon, capsicum, sesame, spearmint and leaf mustard.
[8] The particulate composition of any one of [1] to [6], wherein the oil component (A) comprises 1 to 100 wt% of a bioactive substance, 0 to 99 wt% of a fat and oil, and 0 to 99 wt% of a surfactant (D).
[9] The particulate composition of [8], wherein the surfactant (D) is at least one kind selected from the group consisting of glycerol fatty acid ester, polyglycerin ester, sucrose fatty acid ester, sorbitan fatty acid ester, and propylene glycol fatty acid ester, each having an HLB of not more than 10, and lecithins.
[10] The particulate composition of any one of [1] to [9], wherein the content of the bioactive substance in the particulate composition is 1 - 70 wt%.
[11] The particulate composition of any one of [1] to [10], wherein the volume average particle size is 1 - 1000 µm.
[12] The particulate composition of any one of [1] to [11], wherein the average particle size of the domain(s) is 0.01 to 50 µm .
[13] The particulate composition of any one of [1] to [12], wherein a light-fast ingredient is contained in the composition.
[14] The particulate composition of [13], wherein the light-fast ingredient is at least one kind selected from the group consisting of titanium oxide, food colors, red iron oxide pigment, safflower pigment, annatto pigment, caramel pigment, gardenia pigment, tar pigment and chlorophyll.
[15] An emulsified aqueous solution prepared by dissolving the particulate composition of any one of [1] to [14] in water, wherein a median diameter of the emulsified particles is not more than 2 µm.
[16] A method of producing a particulate composition comprising a bioactive substance, comprising suspending an oil-in-water emulsified composition prepared from an oil component (A) comprising a poorly water-soluble bioactive substance and an aqueous solution comprising a water-soluble excipient comprising a water-soluble polymer as a main component in an oil component (B), and thereafter removing the water in the emulsified composition in the oil component (B).
[17] The method of [16], wherein the oil component (B) comprises 0 - 99.99 wt% of fat and oil and 0.01 - 100 wt% of surfactant (E).
[18] The method of [16], wherein the surfactant (E) is at least one kind selected from the group consisting of glycerol fatty acid ester, polyglycerin ester, sucrose fatty acid ester and sorbitan fatty acid ester, each having an HLB of not more than 10, and lecithins.
[19] The method of any one of [16] to [18], wherein the obtained particulate composition has a sphericity of not less than 0.9.
[20] The method of any one of [16] to [19], wherein the water-soluble polymer is at least one kind selected from the group consisting of gum arabic, ghatti gum, agar, starch, pectin, carrageenan, casein, dried albumen, curdlan, alginic acids, soybean polysaccharide, pullulan, celluloses, xanthan gum, carmellose salt and polyvinylpyrrolidone.
[21] The method of production of any one of [16] to [20], wherein the oil component (A) comprises 1 to 100 wt% of a bioactive substance, 0 to 99 wt% of a fat and oil, and 0 to 99 wt% of a surfactant (D).
[22] The method of production of any one of [16] to [21], wherein the poorly water-soluble bioactive substance is at least one kind selected from the group consisting of oxidized coenzyme Q10, an unsaturated fatty acid, an oil-soluble vitamin, a plant hydrophobic extract ingredient and a carotenoid.
[23] The method of [21], wherein the surfactant (D) is at least one kind selected from the group consisting of glycerol fatty acid ester, polyglycerin ester, sucrose fatty acid ester, sorbitan fatty acid ester and propylene glycol fatty acid ester, each having an HLB of not more than 10, and lecithins.

### Effect of the Invention

The particulate composition of the present invention is a particulate composition comprising a bioactive substance, and having excellent water solubility, workability, tabletability and heat-resistance. A preferable method of producing the particulate composition enables the obtainment of a high recovery rate of 95%, is capable of ameliorating the issue of low recovery rates problematic in the conventional spray drying method and the like, and is advantageous in terms of production costs because there is no need for special equipment as in the spray drying method and the spray cooler method. Furthermore, in a method of production of the present invention, the particle diameter can easily be regulated and hence the handleability is excellent; therefore, a particulate composition having excellent powder characteristics can be obtained without a granulation step as in the spray drying method and the like. In addition, the particulate composition of the present invention can maintain bioactive components, which are easily susceptible to oxidation, stably against oxidation for a long time.

### Brief Description of the Drawings

Figure 1 is a photomicrograph of the particulate composition obtained in Example 1.
Figure 2 is a photomicrograph of the powder obtained in Comparative Example 1.

### Best Mode for Carrying out the Invention

First, the particulate composition of the present invention is described. The particulate composition of the present invention is a particulate composition wherein an oil component (A) comprising a poorly water-soluble bioactive substance is poly-dispersed while forming a domain in a matrix comprising a water-soluble excipient comprising a water-soluble polymer as a main component, and wherein the sphericity is not less than 0.9.

The poorly water-soluble bioactive substance used in the present invention is, for example, a bioactive substance having water solubility of not more than 10 mg/ml, preferably not more than 1 mg/ml, at 25°C.
The bioactive substance used in the present invention may be any substance that is poorly water-soluble as described above and exhibits a useful action in vivo; examples of preferable bioactive substances include oxidized coenzyme Q10, unsaturated fatty acids, oil-soluble vitamins, plant hydrophobic extract ingredients, and carotenoids.

Oxidized coenzyme Q10, which is a bioactive substance to be used in the particulate composition of the present invention, is a compound represented by the following formula (1):

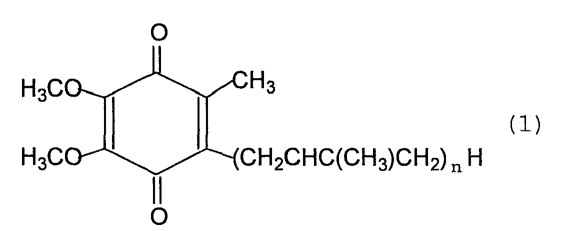

(wherein n=10)

Unsaturated fatty acids, which are bioactive substances to be used in the particulate composition of the present invention, include, but are not limited to, oleic acid, linoleic acid, conjugated linoleic acid, linolenic acid, arachidonic acid, docosahexaenoic acid, docosapentaenoic acid, and eicosapentaenoic acid.
Oil-soluble vitamins, which are bioactive substances to be used in the particulate composition of the present invention, include, but are not limited to, vitamin A, vitamin D, vitamin E, vitamin K, tocotrienol and derivatives thereof.

Plant hydrophobic extract ingredients, which are bioactive substances to be used in the particulate composition of the present invention, include, but are not limited to, hydrophobic ingredients derived from plants or plant processed products in common use for foods, for example, ingredients contained in hydrophobic extracts obtained by extracting licorice, turmeric, perilla, clove, cinnamon, ginger, lemongrass, peppermint, dokudami (Houttuyania cordata), coix seed, rice bran, cornflower, fennel, boxthorn, zanthoxylum, nasturtium, dioscorea, sanryo, kinkaran (Tinospora capillipes), amachazuru (Gynostema pentaphyllum), thuja, hakutouou (Pulsatilla chinensis), parsley, onion, nutmeg, wild-rice, gluten feed, tobiko (konnyaku byproduct powder), paprika, horseradish, lemon, capsicum, sesame, spearmint, or leaf mustard and the like using organic solvents such as ethanol, acetone, and hexane; specifically, polyphenols, terpenes and the like can be mentioned. In the present invention, as plant hydrophobic ingredients, hydrophobic extracts of the above-described plants or plant processed products may be used as is.

Carotenoids, which are bioactive substances to be used in the particulate composition of the present invention, include, but are not limited to, carotenes, xanthophylls, and derivatives thereof; specifically, carotenes are exemplified by α carotene, β carotene, γ carotene, δ carotene, ε carotene, and licopene, and xanthophylls are exemplified by lutein, zeaxanthin, canthaxanthin, fucoxanthin, anthraxanthin, violaxanthin, and astaxanthin.

The matrix in the present invention comprises a water-soluble excipient comprising a water-soluble polymer as a main component and forms a particulate shape.

The water-soluble excipient used in the particulate composition of the present invention comprises a water-soluble polymer as a main component, and may further comprise other components such as a surfactant (C), a saccharide, a yeast cell wall and the like. The term "as a main component" as used herein refers to a state wherein not less than 80 wt% of the water-soluble excipient content, excluding water, is formed with a water-soluble polymer; of course, the water-soluble excipient may consist of a water-soluble polymer only. None of the above-described components of water-soluble excipient are subject to limitation, as long as they are acceptable for use in foods, cosmetics, or pharmaceuticals, with preference given to those acceptable for foods.

While the content of the water-soluble polymer in the particulate composition is not limited by the kind of the included bioactive substance, it is preferably within the range of 20-99 wt%, more preferably 30-95 wt%, still more preferably 40-90 wt%.
As the above-mentioned water-soluble polymer, for example, gum arabic, ghatti gum, guar gum, agar, starch, pectin, carrageenan, casein, casein compound, dried albumen, curdlan, alginic acids, soybean polysaccharides, pullulan, celluloses, xanthan gum, carmellose salt (carmellose sodium, carmellose calcium and the like), higher fatty acid sugar ester, tragacanth, water-soluble polymer containing amino acid and/or sugar and the like as main components such as milk and the like, polyvinylpyrrolidone and the like can be used singly or in a mixture of two or more kinds thereof. Of these, gum arabic, ghatti gum, guar gum, agar, starch, pectin, carrageenan, casein, dried albumen, curdlan, alginic acids, soybean polysaccharides, pullulan, celluloses, xanthan gum, carmellose salt and polyvinylpyrrolidone are preferable. Gum arabic, ghatti gum, and soybean polysaccharides are more preferably used and gum Arabic or ghatti gum is most preferably used because a particulate composition simultaneously having excellent water solubility of aqueous solution during production, workability, tabletability and heat-resistance can be obtained, or the included bioactive substance can be stably maintained against oxidation and the like.

While the above-mentioned surfactant (C) that may be contained in the above-mentioned water-soluble excipient is not particularly limited as long as it is acceptable for food, cosmetic and pharmaceutical product, one particularly acceptable for food is preferable. For example, glycerol fatty acid esters, sucrose fatty acid esters, sorbitan fatty acid esters, lecithins and saponins can be used. It is needless to say that they can be used alone or in a mixture of two or more kinds thereof in the present invention.

As the aforementioned glycerol fatty acid esters, for example, fatty acid and organic acid esters of monoglycerol, polyglycerol fatty acid esters, polyglycerin condensed ricinoleate and the like can be mentioned.
As the fatty acid and organic acid esters of monoglycerol, for example, stearic acid and citric acid ester of monoglycerol, stearic acid and acetic acid ester of monoglycerol, stearic acid and succinic acid ester of monoglycerol, caprylic acid and succinic acid ester of monoglycerol, stearic acid and lactic acid ester of monoglycerol, stearic acid and diacetyltartaric acid ester of monoglycerol and the like can be mentioned.
As the polyglycerol fatty acid ester, for example, one having an average degree of polymerization of polyglycerin of 2 - 10, wherein the constituent fatty acid has 6 to 22 carbon atoms, can be mentioned.
As the aforementioned polyglycerin condensed ricinoleate, for example, one having an average degree of polymerization of polyglycerin of 2 - 10, wherein the average degree of condensation of polyricinoleic acid (average number of condensation of ricinoleic acid) is 2 to 4, can be mentioned.

As the aforementioned sucrose fatty acid esters, one wherein one or more hydroxyl groups of sucrose is/are each esterified with fatty acid having 6 to 18, preferably 6 to 12, carbon atoms can be mentioned.

Examples of the aforementioned sorbitan fatty acid esters include sorbitans wherein one or more hydroxyl groups thereof are esterified by fatty acids having 6 to 18, preferably 6 to 12, carbon atoms, and polyoxyethylene sorbitan fatty acid esters wherein polyoxyethylene is added to hydroxyl groups of sorbitans.

As the aforementioned lecithins, for example, egg-yolk lecithin, purified soybean lecithin, phosphatidylcholine, phosphatidylethanolamine, phosphatidylserine, sphingomyelin, dicetyl phosphate, stearylamine, phosphatidylglycerol, phosphatidic acid, phosphatidylinositolamine, cardiolipin, ceramide phosphorylethanolamine, ceramide phosphoryl glycerol, enzymatically decomposed lecithin and a mixture thereof and the like can be mentioned.

As the aforementioned saponins, for example, enju saponin, quillaja saponin, soybean saponin, yucca saponin and the like can be mentioned.

Of the above-mentioned surfactant (C), surfactant (C) is preferably a hydrophilic surfactant and, for example, a surfactant having an HLB of not less than 4, generally not less than 6, preferably not less than 8, more preferably not less than 9.5, more preferably not less than 11 can be used because an oil component containing a bioactive substance can be emulsified stably, and a particulate composition simultaneously having excellent water solubility, workability, and tabletability, which is the object of the present invention, can be obtained.
As such surfactant, specifically, fatty acid and organic acid esters of monoglycerol such as stearic acid and citric acid ester of monoglycerol, stearic acid and diacetyltartaric acid ester of monoglycerol and the like; polyglycerol fatty acid esters such as triglycerol monolaurate, triglycerol monomyristate, triglycerol monooleate, triglycerol monostearate, pentaglycerol monomyristate, pentaglycerol trimyristate, pentaglycerol monooleate, pentaglycerol trioleate, pentaglycerol monostearate, pentaglycerol tristearate, hexaglycerol monocaprylate, hexaglycerol dicaprylate, hexaglycerol monolaurate, hexaglycerol monomyristate, hexaglycerol monooleate, hexaglycerol monostearate, decaglycerol monolaurate, decaglycerol monomyristate, decaglycerol monooleate, decaglycerol monopalmitate, decaglycerol monostearate, decaglycerol distearate and the like; polyglycerin condensed ricinoleate such as tetraglycerol condensed ricinoleate, pentaglycerol condensed ricinoleate, hexaglycerol condensed ricinoleate, diglycerol condensed ricinoleate and the like; sorbitan fatty acid esters such as sorbitan monostearate, sorbitan monooleate and the like; sucrose fatty acid esters such as sucrose palmitate, sucrose stearate and the like; lecithins such as soybean lecithin, egg-yolk lecithin, enzymatically decomposed lecithin and the like; and saponins such as enju saponin, quillaja saponin, soybean saponin, yucca saponin and the like can be mentioned.

The sugar that may be contained in the water-soluble excipient is not particularly limited as long as it is acceptable for food and, for example, monosaccharides such as glucose, fructose, galactose, arabinose, xylose, mannose and the like; disaccharides such as maltose, sucrose, trehalose, lactose and the like; oligosaccharides such as fructooligosaccharide, soybean oligosaccharide, galactooligosaccharide, xylo-oligosaccharide, cyclodextrine and the like; sugar alcohols such as sorbitol, maltitol, erythritol, lactitol, xylitol and the like; and the like can be preferably used.

Yeast cell walls that may be contained in the water-soluble excipient include beer yeast cell walls and the like.

When the bioactive substance to be used in the present invention is easily oxidized, for example, when unsaturated fatty acids or astaxanthin is used as a bioactive substance, water-soluble polymer and sugar are preferably used in combination as the water-soluble excipient. It is more preferable to combine gum arabic as the water-soluble polymer and sucrose or dextrin as the sugar. When a water-soluble polymer and sugar are used in combination, the weight ratio of water-soluble polymer and sugar is not particularly limited. The weight of the water-soluble polymer relative to the total weight of water-soluble polymer and sugar is generally not less than 25%, preferably not less than 40%, more preferably not less than 50%, particularly preferably not less than 60%, and generally not more than 99%, preferably not more than 95%, more preferably not more than 90%, particularly preferably not more than 85%. When the bioactive substance to be used is stable to oxidation, the weight proportion mentioned above may not apply, and a water-soluble polymer such as gum arabic and the like can be preferably used alone as a water-soluble excipient.

In the particulate composition of the present invention, the oil component (A) comprising a bioactive substance may be (1) the bioactive substance alone or (2) a mixture of the bioactive substance and a fat and oil and/or a surfactant (D). If the fat and oil component (A) is a mixture of the bioactive substance and a fat and oil and/or a surfactant (D), it is preferable that the bioactive substance be dissolved or visually uniformly mixed in the fat and oil and/or the surfactant (D) to obtain an oil component under the temperature conditions during manufacturing (particularly in the emulsification step). For maintaining a high content of a bioactive substance in oil component (A), the above-mentioned (1) is preferable.

The fats and oils to be used when oil component (A) is the aforementioned (2) are not particularly limited and, for example, may be natural fats and oils from plants and animals, synthetic fats and oils or processed fats and oils. More preferably, one acceptable for food, cosmetic or pharmaceutical agent is used. Examples of vegetable oil include coconut oil, palm oil, palm kernel oil, flaxseed oil, camellia oil, brown rice germ oil, canola oil, rice oil, peanuts oil, corn oil, wheat germ oil, soy bean oil, perilla oil, cottonseed oil, sunflower kerel oil, kapok oil, evening primrose oil, shea butter, sal butter, cacao butter, sesame oil, safflower oil, olive oil, pomegranate oil, bitter gourd oil and the like, and examples of animal fats and oils include lard, milk fat, fish oil, beef fat and the like. Furthermore, fats and oils obtained by processing them such as by fractionation, hydrogenation, transesterification (e.g., hydrogenated oil) and the like are also included. It is needless to say that medium-chain triglyceride (MCT) and the like can also be used. A mixture thereof may be used. As the medium chain triglyceride, for example, triglyceride wherein fatty acid has 6 to 12, preferably 8 to 12, carbon atoms can be mentioned.

Of the above-mentioned fats and oils, vegetable fats and oils, synthetic fats and oils and processed fats and oils are preferable from the aspects of handleability, odor and the like. For example, coconut oil, palm oil, palm kernel oil, canola oil, rice oil, soy bean oil, cottonseed oil, safflower oil, olive oil, MCT and the like can be mentioned.

Examples of the surfactant (D) used in the case (2) of fat and oil component (A) include, but are not limited to, glycerol fatty acid esters, polyglycerin esters, sucrose fatty acid esters, sorbitan fatty acid esters and propylene glycol fatty acid esters, with preference given to those having HLB values of not more than 10 or lecithins.

As such glycerol fatty acid esters, for example, monoglycerides and diglycerides wherein fatty acid has 6 to 18, preferably 6 to 12, carbon atoms can be mentioned.
As the polyglycerin esters, for example, polyglycerin comprising polyglycerin having a polymerization degree of 2 to 10 as a main component, wherein one or more hydroxyl groups of polyglycerin is/are esterified with fatty acid having 6 to 18, preferably 6 to 12, carbon atoms can be mentioned.
As the sucrose fatty acid esters, one wherein one or more hydroxyl groups of sucrose is/are esterified with fatty acid having 6 to 18, preferably 6 to 12, carbon atoms can be mentioned.
As the sorbitan fatty acid esters, one wherein one or more hydroxyl groups of sorbitan is/are esterified with fatty acid having 6 to 18, preferably 6 to 12, carbon atoms, and polyoxyethylene sorbitan fatty acid esters wherein polyoxyethylene is added to hydroxyl groups of sorbitans can be mentioned.
As the propylene glycol fatty acid esters, for example, monoglycerides and diglycerides wherein fatty acid has 6 to 18, preferably 6 to 12, carbon atoms can be mentioned.
As the lecithins, for example, egg-yolk lecithin, purified soybean lecithin, phosphatidylcholine, phosphatidylethanolamine, phosphatidylserine, sphingomyelin, dicetyl phosphate, stearylamine, phosphatidylglycerol, phosphatidic acid, phosphatidylinositolamine, cardiolipin, ceramide phosphorylethanolamine, ceramide phosphoryl glycerol, enzymatically decomposed lecithin, and a mixture thereof and the like can be mentioned.

Of the above-mentioned surfactant (D), a hydrophilic surfactant is preferable for obtaining a particulate composition simultaneously having excellent water solubility, workability, and tabletability and, for example, a surfactant having an HLB of not more than 9, preferably not more than 8, more preferably not more than 6, still more preferably less than 5 can be used.

As such surfactant, specifically, a mixture of one or more kinds selected from monoglycerol monofatty acid esters such as monoglycerol monostearate, monoglycerol monooleate, monoglycerol monomyristate, monoglycerol monocaprylate, monoglycerol monolaurate, monoglycerol monobehenate, monoglycerol monoerucate and the like; monoglycerol difatty acid esters such as monoglycerol distearate, monoglycerol dioleate, monoglycerol dicaprylate, monoglycerol dilaurate and the like; fatty acid and organic acid esters of monoglycerol such as stearic acid and citric acid ester of monoglycerol, stearic acid and acetic acid ester of monoglycerol, hydrogenated coconut oil and acetic acid ester of monoglycerol, stearic acid and succinic acid ester of monoglycerol, caprylic acid and succinic acid ester of monoglycerol, stearic acid and lactic acid ester of monoglycerol, stearic acid and diacetyltartaric acid ester of monoglycerol and the like; monoglycerol fatty acid esters obtained using various fats and oils such as hydrogenated beef tallow and fatty acid esters of monoglycerol, hydrogenated canola oil and fatty acid esters of monoglycerol, hydrogenated soybean oil and fatty acid esters of monoglycerol, cottonseed oil and fatty acid esters of monoglycerol, safflower oil and fatty acid esters of monoglycerol and the like; polyglycerol fatty acid esters such as ester of polyglycerin having an average polymerization degree of 2 - 10 and fatty acid having 6 to 22 carbon atoms and the like; glycerol fatty acid esters such as polyglycerol fatty acid esters polyglycerin condensed ricinoleate (ester of polyglycerin having an average polymerization degree of 2 - 10 and polyricinoleic acid having a condensation degree of 2 - 4 and the like); propylene glycol fatty acid esters such as propylene glycol monostearate, propylene glycol monooleate, and propylene glycol monolaurate and the like; sucrose fatty acid esters such as sucrose oleate, sucrose palmitate, sucrose stearate, sucrose laurate, sucrose myristate, sucrose behenate and the like; sorbitan fatty acid esters such as sorbitan distearate, sorbitan tristearate, sorbitan sesquioleate, sorbitan dioleate, and sorbitan trioleate and the like; and lecithins such as soybean lecithin, egg-yolk lecithin, enzymatically decomposed lecithin and the like can be mentioned.

Of these, preferred is a mixture of one or more kinds selected from glycerol fatty acid esters, more preferred is a mixture of one or more kinds selected from monoglycerol monofatty acid esters, monoglycerol difatty acid esters, fatty acid and organic acid esters of monoglycerol (particularly fatty acid and acetic acid esters of monoglycerol, hydrogenated coconut oil and acetic acid ester of monoglycerol), polyglycerol fatty acid esters (particularly diglycerol monofatty acid esters) and polyglycerin condensed ricinoleate (particularly ester of polyglycerin having an average degree of polymerization of 2 - 10 and polyricinoleic acid having a condensation degree of 2 - 4), more preferred is fatty acid and organic acid esters of monoglycerol (particularly fatty acid and acetic acid esters of monoglycerol, hydrogenated coconut oil and acetic acid esters of monoglycerol). Specific examples of the above-mentioned fatty acid and acetic acid esters of monoglycerol, hydrogenated coconut oil and acetic acid esters of monoglycerol include 50% acetylated product of monoglycerol monostearate, completely acetylated product of hydrogenated coconut oil monoglyceride. The above-mentioned surfactant can be used alone or in a mixture of two or more kinds thereof.

Besides the above-mentioned, the oil component (A) in the present invention may contain, according to various objects, an oil-soluble component such as solid fat and oil, fatty acid and ester derivatives thereof and the like.

As the aforementioned solid fat and oil, for example, wax for food such as bees wax, vegetable wax, candelilla wax, rice bran wax, carnauba wax, snow wax and the like can be mentioned.

Examples of the aforementioned fatty acid and ester derivatives thereof include, but are not limited to, saturated fatty acids such as caprylic acid, capric acid, lauric acid, myristic acid, palmitic acid, stearic acid, and behenic acid; unsaturated fatty acids such as oleic acid, linoleic acid, conjugated linoleic acid, linolenic acid, punicic acid, docosahexaenoic acid, docosapentaenoic acid, and eicosapentaenoic acid; special fatty acids having an SS bond in the molecular structure thereof, such as α lipoic acid, and esters thereof, for example, methyl esters and ethyl esters thereof, and the like. The final content of oil component (A) containing a poorly water-soluble bioactive substance in the particulate composition of the present invention is not particularly limited by the kind of the bioactive substance. However, it is preferably within the range of 1-80 wt%, more preferably 5-70 wt%, still more preferably 10-60 wt%. When oxidized coenzyme Q10 is used as a bioactive substance, the final content of oil component (A) in the particulate composition is particularly preferably 40-60 wt%. When unsaturated fatty acid, oil-soluble vitamin or a plant hydrophobic extract ingredient is used as a bioactive substance, the final content of oil component (A) in the particulate composition is particularly preferably 10-30 wt%.

In the particulate composition of the present invention, the oil component (A) comprising a poorly water-soluble bioactive substance preferably comprises 1 to 100 wt% of a bioactive substance, 0 to 99 wt% of a fat and oil, and 0 to 99 wt% of a surfactant (D), more preferably comprising 10 to 100 wt% of a bioactive substance, 0 to 90 wt% of a fat and oil, and 0 to 90 wt% of a surfactant, still more preferably comprising 25 to 100 wt% of a bioactive substance, 0 to 75 wt% of a fat and oil, and 0 to 75 wt% of a surfactant (D), most preferably comprising 50 to 100 wt% of a bioactive substance, 0 to 50 wt% of a fat and oil, and 0 to 50 wt% of a surfactant (D). This does not apply, however, if the solubility of the bioactive substance in the fat and oil and/or the surfactant (D) is low. If the content of the bioactive substance in the oil component (A) is less than 1 wt%, the content of the bioactive substance in the finally obtained particulate composition decreases, so that a large amount of the particulate composition must be taken when the specified amount of the bioactive substance is orally administered, and this is undesirable.

The particulate composition of the present invention may comprise a light-fast ingredient added thereto for the purpose of stabilizing a bioactive substance or any other ingredient that is unstable to light. The light-fast ingredient may be any ingredient useful in foods, quasi-drugs, or pharmaceuticals. For example, colorants for soft capsules and hard capsules for oral administration are preferable; specifically, pigments such as titanium oxide, food colors, red iron oxide pigment, safflower pigment, annatto pigment, caramel pigment, gardenia pigment, tar pigment, and chlorophyll, ultraviolet absorbents and the like can be mentioned. The light-fast ingredient can be added to, and contained in, the water-soluble excipient if it is hydrophilic, and the oil component (A) if it is oleophilic. The amount of light-fast ingredient added is not subject to limitation, as long as the disintegrability or solubility of the particulate composition is not affected; for example, the amount is in the range from 0.01 to 10.0 wt%, preferably 0.1 to 5.0 wt%, relative to the particulate composition. If the amount added is less than 0.01 wt%, possibly no light stability effect is obtained; if the amount added exceeds 10.0 wt%, the disintegrability and solubility of the particulate composition are possibly affected.

In addition, the particulate composition of the present invention may comprise additives and water-soluble active ingredients useful for a wide variety of purposes in foods, cosmetics, and pharmaceuticals, added according to the respective purposes, as long as the properties thereof are not affected.

For example, in addition to the above-mentioned compounds, excipients such as crystalline cellulose, calcium phosphate, calcium sulfate and the like, disintegrants such as calcium citrate, calcium carbonate, sodium hydrogencarbonate, dextrin, crystalline cellulose, carboxymethylcellulose, tragacanth, alginic acid and the like, lubricants such as talc, magnesium stearate, polyethylene glycol, silica, hydrogenated oil and the like, antiblocking agents such as stearic acid, talc, light anhydrous silicic acid, hydrated silicon dioxide and the like, absorption promoters such as higher alcohols, higher fatty acids and the like, solubilizing agents such as fumaric acid, succinic acid, malic acid and the like, stabilizers such as benzoic acid, sodium benzoate, ethyl p-oxybenzoate, bees wax and the like can be used as additives.

Examples of the water-soluble active ingredients include, but are not limited to, water-soluble vitamins, amino acids, organic acids, peptides, proteins, nucleic acids, water-soluble polyphenols, salts, and animal/plant water-soluble extracts.

Examples of the water-soluble vitamins include vitamin C, vitamin Bs, folic acid, nicotinic acid, nicotinic acid amide, pantothenic acid, pyrroloquinolinequinone, and isomers and derivatives thereof.

Examples of the amino acids include alanine, 3-alanine, arginine, asparagine, aspartic acid, cysteine, N-acetylcysteine, selenocysteine, cystine, glutamine, glutamic acid, glycine, histidine, isoleucine, leucine, lysine, pyrrololysine, hydroxylysine, methionine, phenylalanine, proline, hydroxyproline, serine, O-phosphoserine, threonine, tryptophan, tyrosine, valine, thyroxine, desmosine, ornithine, creatine, γ-aminobutyric acid, theanine, taurine, and isomers and derivatives thereof. L-carnitine, a special amino acid, and pharmacologically acceptable salts thereof, such as tartrates and fumarates, acetyl-L-carnitine, and propionyl-L-carnitine are also suitable. It is also suitable to use peptides of two or more of these amino acids bound together; examples include, but are not limited to, cysteine peptides such as glutathione, soybean peptide, sesame peptide, silk peptide, sardine peptide, collagen peptide, casein phosphopeptide, proteins such as wheat protein, soybean protein, milk serum protein and egg protein, nucleic acids such as deoxyribonucleic acid, ribonucleic acid, guanylic acid and inosinic acid. Examples of organic acids other than amino acids include, but are not limited to, citric acid, malic acid, succinic acid, catechinic acid, picric acid, fumaric acid, maleic acid, and tartaric acid.

The term polyphenol generically refers to a group of plant ingredients having a plurality of phenolic hydroxyl groups (hydroxy groups) in the molecular structure thereof; these ingredients are water-soluble (some are oil-soluble) substances having excellent antioxidant power, contained in most plants as pigments or bitter tastes. Examples of water-soluble polyphenols include, but are not limited to, grape polyphenol, pine tree bark polyphenol, apple polyphenol, cacao polyphenol, and green tea polyphenol. These polyphenols occur as multi-ingredient systems; specifically, flavonoids are exemplified by isoflavons such as genistin, daidzein, and puerarin; flavonols such as quercetin, kaempferol, myricetin, and rutin; flavanones such as hesperidin, naringenin, and naringin; flavanols such as anthocyanines which are glucosides of cyanidin, delphinidin, malvidin, peonidin, petunidine, and the like, epicatechin, epigallocatechin, epicatechin gallate, epigallocatechin gallate, and theaflavine; and flavons such as chrysin, apigenin, and luteolin. Lignans such as sesamin, sesamolin, sesaminol, and sesamol, or chlorogenic acid, gallic acid, ellagic acid, galangin, fisetin and the like are also suitable. Furthermore, minerals can be added as other useful ingredients; examples include, but are not limited to sodium, calcium, magnesium, zinc, iron, copper, selenium, chromium, manganese, iodine, molybdenum, and salts thereof.

The average particle size of the domain formed by the oil component (A) containing a poorly water-soluble bioactive substance in the particulate composition of the present invention is not particularly limited as long as the object of the present invention can be achieved. When the average particle size of the domain formed is large, absorbability of the particulate composition may decrease. Thus, the average particle size is generally not more than 50 µm, preferably not more than 20 µm, more preferably not more than 15 µm, particularly preferably not more than 10 µm. On the other hand, when the average particle size of the domain is small, problems occur in that excess water-soluble excipient is needed to maintain stability of emulsion droplet during the production process, excess load is applied to an emulsification apparatus and the like. Thus, the average particle size is generally 0.001 µm, preferably not less than 0.005 µm, more preferably not less than 0.01 µm, particularly preferably not less than 0.1 µm.

The average particle size of the domain formed by the oil component (A) comprising a bioactive substance can be determined by breaking the particulate composition into hemispheres, and examining the breakage faces on electron microscopic images by image analysis.

The content of the poorly water-soluble bioactive substance in the particulate composition of the present invention is preferably in the range from 1 to 70 wt%, more preferably in the range from 5 to 60 wt%, still more preferably in the range from 10 to 50 wt%, most preferably in the range from 20 to 40 wt%, relative to the weight of the particulate composition. If the content of the poorly water-soluble bioactive substance in the particulate composition is less than 1 wt%, a large amount of the particulate composition must be taken when the specified amount of the bioactive substance is orally administered, and this is undesirable.

In the particulate composition of the present invention, an oil component (A) containing a poorly water-soluble bioactive substance is polydispersed, forming preferably not less than 5 domains, more preferably not less than 1,000, more preferably not less than 10,000, particularly preferably not less than 100,000 in the matrix containing a water-soluble excipient. While the upper limit is not particularly limited, it is generally about 1,000,000,000.

If the number of domains in the matrix comprising the water-soluble excipient is less than five, the content of the bioactive substance in the finally obtained powder composition decreases, and a large amount of the particulate composition must be taken when the specified amount of the bioactive substance is orally administered, and this is undesirable.

In the particulate composition of the present invention, the oil component (A) comprising a bioactive substance is poly-dispersed while forming a domain in a water-soluble excipient matrix, and the structure thereof is controlled to have a sphericity of not less than 0.9. Hence, in the present invention, the sphericity of the particulate composition must not be less than 0.9. The sphericity is preferably not less than 0.94. As the sphericity of the particulate composition increases, the powder characteristics of the particulate composition improve, the workability and tabletability improve, and furthermore, the stability to oxidation of the included bioactive substance improves.

The sphericity of the particulate composition can be determined by, for example, taking electron photomicrographs of the subject particulate composition, and examining the images using the image analysis software WinROOF Ver. 3.30 and the like to determine the ratio of the diameter of the circle having the same area and the diameter of the least circle circumscribed.

Furthermore, in the particulate composition of the present invention, the surface roughness (Ra) thereof is preferably as small as possible. As the surface roughness (Ra) of the particulate composition decreases, the total surface area per unit weight of the particulate composition decreases; accordingly, not only the particulate composition becomes less likely to undergo the effects of oxidation reactions by oxygen molecules in the air and the like, which are estimated to proceed from the particle surface, but also the powder characteristics (e.g., fluidity) improve. The surface roughness (Ra) of particles can be determined as, for example, arithmetic mean surface roughness (Ra) as specified by JIS B 0601-1994. The surface roughness here is considered to be in an opposite relationship with the above-mentioned sphericity, where the sphericity is high, the surface roughness tends to be small. When, in the particulate composition of the present invention, the included bioactive substance is particularly easily oxidized, a higher absolute specific gravity of the water-soluble excipient matrix is more preferable, which is concretely preferably not less than 1.25. By setting a high absolute specific gravity of the water-soluble excipient matrix, the oxidation stability of the included bioactive substance can be enhanced. For setting the absolute specific gravity of the water-soluble excipient matrix to not less than 1.25, a preferable production method to be mentioned later can be employed. When a known spray cooler method or liquid hardening method is used, the absolute specific gravity of the obtained microcapsule does not become very high and is generally less than 1.25.

In the present invention, even if an ingredient that is crystalline at normal temperature, such as oxidized coenzyme Q10, is used as the poorly water-soluble bioactive substance, the particulate composition obtained by the method of production described below has a feature that it is unlikely to crystallize even if stored at normal temperature after manufacturing because the oil component (A) comprising a bioactive substance, in a molten state, is enclosed in microcapsules surrounded by particles of a water-soluble excipient. For example, if a particulate composition comprising oxidized coenzyme Q10 is produced by the following method of production, even after storage at 25°C in the air for 30 days following manufacturing, not less than 10 wt% of the oxidized coenzyme Q10 in the particulate composition does not crystallize. Usually, bioactive substances (e.g., oxidized coenzyme Q10) in a non-crystalline state or molten state are more likely to undergo emulsification by surfactant components that coexist in vivo or in the particulate composition in the stomach or intestine than do bioactive substances (e.g., oxidized coenzyme Q10) in a crystalline state; as a result, bioactive substances in a non-crystalline state or molten state are more likely to be absorbed at increased rates from the gastrointestinal tract than do bioactive substances in a crystalline state; as a result, a preferable particulate composition of the present invention is thought to have high oral absorbability, which is one of the objects thereof.

The volume average particle size of the particulate composition of the present invention is not subject to limitation, as long as an object of the present invention can be accomplished, and this diameter is preferably in the range from 1 to 1000 µm, more preferably 10 to 800 µm, most preferably 50 to 700 µm. If the volume average particle size of the particulate composition is smaller than 1 µm, recovery as a powder becomes difficult, and this is undesirable. The upper limit of the volume average particle size is not particularly limited as long as the high stability and high absorbability of a poorly water-soluble bioactive substance, which is the object of the present invention, can be maintained. For easy processing into food, pharmaceutical product, cosmetic and the like, it is preferably not more than 1000 µm.
The volume average particle size can be measured using, for example, an ethanol solvent in a laser diffraction scattering type particle size distribution measurement apparatus (Microtruck MT3000II manufactured by NIKKISO CO., LTD.).

Next, a preferable method of producing the particulate composition of the present invention, comprising a bioactive substance, is described. However, the following is not to be construed as limiting the scope of the invention, provided that a similar particulate composition is obtained by any other method of production.

The particulate composition of the present invention, comprising a bioactive substance, is preferably produced by a method comprising suspending an oil-in-water emulsified composition prepared from an oil component (A) comprising a poorly water-soluble bioactive substance and an aqueous solution comprising a water-soluble excipient comprising a water-soluble polymer as a main component in an oil component (B), and then removing the water in the oil component (B).

In the above-mentioned production method, the water-soluble excipient is preferably used in the form of an aqueous solution dissolved in water, where the concentration is free of any particular limitation. It is preferable to handle at a concentration at which the viscosity of aqueous solution does not exceed 1 Poise, since the transferring property and the like can be ensured. Specific examples and preferable examples of the water-soluble excipient here are the same as those recited in the above-mentioned explanation of the particulate composition.

In the above-described method of production, if a bioactive substance is used alone as the oil component (A), or if a bioactive substance whose solubility in fats and oils at normal temperature is low is used, the most convenient and preferable method for preparing the oil component (A) comprising a poorly water-soluble bioactive substance is to warm the bioactive substance to a temperature at which it melts or dissolves in the fat and oil, add a fat and oil or/and a surfactant (D) and the like as required, and blend them by stirring and the like, but this is not to be construed as a limiting factor. For example, if oxidized coenzyme Q10 is used as the bioactive substance, oxidized coenzyme Q10 molten above 50°C as is or as blended with a fat and oil or/and a surfactant (D) and the like added as required, is used as the oil component (A). If the poorly water-soluble bioactive substance is soluble in the fat and oil at normal temperature, a mixture of the bioactive substance and a fat and oil or/and a surfactant (D) and the like may be used as the oil component (A) without warming.

Specific examples and preferable examples of oil component (A) here are the same as those recited in the above-mentioned explanation of the particulate composition.

Next, in the method of production of the present invention, an oil-in-water emulsified composition is prepared from the above-described oil component (A) comprising a bioactive substance and an aqueous solution comprising a water-soluble excipient. The most convenient and preferable method of preparing the oil-in-water emulsified composition is to add the oil component (A) comprising a bioactive substance to an aqueous solution comprising a water-soluble excipient, and finely disperse and emulsify the oil component (A) to the desired average particle size using a commonly known mechanical emulsifier such as a high-pressure homogenizer. If warming is required at the time of preparation of the oil component (A), it is preferable that warming be performed also in the emulsification step.

In addition to these methods, it is also possible to add a bioactive substance, or another oil component as required, to an aqueous solution comprising a water-soluble excipient, and warm the solution as required, so as to convert the bioactive substance or the other oil component into oil drops in the water-soluble excipient aqueous solution, and then emulsify the same, but these are not to be construed as limiting the present invention.

In the method of production of the present invention, the emulsified particle diameter of the oil component (A) comprising a bioactive substance in the above-described oil-in-water emulsified composition is preferably in the range from 0.01 to 50 µm, more preferably in the range from 0.01 to 20 µm, most preferably in the range from 0.01 to 10 µm. If the average particle size of the oil component (A) in the oil-in-water emulsified composition exceeds 50 µm, the oral absorbability of the particulate composition obtained tends to decrease. If the average particle size of the oil component (A) of the oil-in-water emulsified composition is smaller than 0.01 µm, it tends to be difficult to maintain the stability of emulsion droplets in the manufacturing process. By controlling the particle diameter of emulsion droplets in this step, the domain particle diameter in the particulate composition obtained can be controlled.

The above-mentioned emulsified particle diameter of oil component (A) in the oil-in-water emulsified composition can be measured using a commercially available laser diffraction scattering type particle size distribution measurement apparatus.

In the method of production of the present invention, the step for preparing an oil-in-water emulsified composition from the oil component (A) comprising a bioactive substance and an aqueous solution comprising a water-soluble excipient is preferably performed under temperature conditions that make the oil component (A) in a solution state or homogeneous, and the temperature is normally in the range from 50 to 100°C. The upper limit of the temperature in the emulsification step varies depending on apparatus specifications, and is normally preferably not more than 120°C.

In the production method of the present invention, the above-mentioned oil-in-water emulsified composition is mixed with a different oil component (B), and the oil-in-water emulsified composition is suspended in oil component (B) to a desired particle size, whereby an O/W/O emulsion can be produced. The above-mentioned mixing operation is, for example, most conveniently and preferably performed by adding an oil-in-water emulsified composition containing a bioactive substance to oil component (B) heated in advance to not less than 50°C. However, the method is not limited to this. The size of the particles suspended in the oil-in-water emulsified composition in oil component (B) can be adjusted by applying shear to the mixture such as stirring. The temperature of oil component (B) during preparation of the mixture is preferably generally within the range of 50 - 100°C to prevent rapid evaporation of water.

In the method of production of the present invention, the blending ratio of the oil-in-water emulsified composition and the oil component (B) is not subject to limitation, but the % by weight of the oil-in-water emulsified composition in the mixture of the oil-in-water emulsified composition and the oil component (B) is preferably 1 to 70 wt%, more preferably 10 to 60 wt%, most preferably 15 to 55 wt%. The % of weight of the oil-in-water emulsified composition in the mixture of the oil-in-water emulsified composition and the oil component (B) is preferably not less than 1 wt% in view of the production efficiency and preferably not more than 70 wt% in consideration of suspensibility and the like.

In the production method of the present invention, the above-mentioned O/W/O emulsion is afforded and then water is removed from the oil-in-water emulsified composition suspended in oil component (B). For removal of water from the oil-in-water emulsified composition, for example, the composition is heated to not less than 80°C, preferably not less than 100°C, under atmospheric pressure to evaporate water. Alternatively, a method including setting the temperature to a temperature not less than the boiling point of water (at the corresponding pressure), under any reduced pressure, and evaporating water and the like can be mentioned, but the method is not limited thereto.

In the present invention, a mixture of fat and oil and surfactant (E) is generally used as oil component (B). Depending on the kind of surfactant (E) used, surfactant (E) may also be used alone as oil component (B) without using fat and oil. While the contents of fat and oil and surfactant (E) in oil component (B) are not particularly limited, surfactant (E) is preferably adjusted to be within the range of 0.01-100 wt% relative to 0-99.99 wt% of fat and oil. When the amount of surfactant (E) to be added is lower than 0.01 wt%, the suppressive effect on the agglomeration of droplets of the oil-in-water emulsified composition during drying tends to be difficult to achieve.

The fat and oil used in the oil component (B) in the method of production of the present invention is not subject to limitation, as long as it is a fat and oil capable of suspending the above-described oil-in-water emulsified composition, and it may be, for example, a natural fat and oil of animal/plant origin, and may be a synthetic fat and oil or a processed fat and oil. More preferably, the fat and oil is a fat and oil acceptable for use in foods, cosmetics or pharmaceuticals. Examples of plant fats and oils include coconut oil, palm oil, palm kernel oil, linseed oil, camellia oil, brown rice germ oil, rapeseed oil, rice oil, peanut oil, corn oil, wheat germ oil, soybean oil, perilla oil, cottonseed oil, sunflower seed oil, kapok oil, evening primrose oil, shea butter, sal butter, cacao butter, sesame oil, safflower oil, olive oil and the like; examples of animal fats and oils include lard, milk fat, fish oil, beef tallow and the like; furthermore, fats and oils processed therefrom by fractionation, hydrogenation, transesterification (e.g., hydrogenated oil) can be mentioned. Of course, medium-chain fatty acid triglycerides (MCT) can also be used. A mixture thereof may be used.

Examples of the medium-chain fatty acid triglyceride include triglyceride wherein fatty acid has 6 to 12 carbon atoms, preferably 8 to 12 carbon atoms

Of the above-mentioned fats and oils, vegetable fats and oils, synthetic fats and oils and processed fats and oils are preferable from the aspects of handleability, odor and the like. For example, coconut oil, palm oil, palm kernel oil, canola oil, rice oil, soy bean oil, cottonseed oil, safflower oil, olive oil, MCT and the like can be used.

In the method of production of the present invention, a surfactant (E) can be used as a component of the oil component (B). The droplet of the oil-in-water emulsified composition gradually comes to have greater adhesiveness as the progress of drying, and particles tend to easily agglomerate with each other. However, in the co-presence of surfactant (E) in oil component (B), agglomeration of oil-in-water emulsified composition droplets with increased adhesiveness during drying is drastically reduced and, as a result, the recovery rate of particulate composition having a desired volume average particle size can preferably be improved strikingly.

While the content of surfactant (E) in oil component (B) is free of any particular limitation, the wt% of surfactant (E) relative to oil component (B) is generally not less than 0.001 wt%, preferably not less than 0.005 wt%, more preferably not less than 0.01 wt%, from the aspect of suppression of agglomeration during drying of the oil-in-water emulsified composition droplets and the like. While the upper limit is not particularly limited, it is generally not more than 95 wt%, preferably not more than 80 wt%, more preferably not more than 60 wt%, from the aspect of flowability of oil component (B), removal of surfactant (E) and the like.

The above-mentioned surfactant (E) is not particularly limited as long as it is acceptable to be used for food, cosmetic or pharmaceutical product. A surfactant acceptable for food is particularly preferable and, for example, surfactants having an HLB of not more than 10 such as glycerol fatty acid esters, polyglycerol esters, sucrose fatty acid esters, sorbitan fatty acid esters and the like, and lecithins can be used. Needless to say, they may be used alone or in a mixture of two or more kinds thereof in the present invention.

Examples of glycerol fatty acid esters include monoglycerides and diglycerides wherein fatty acid has 6 to 18, preferably 12 to 18, carbon atoms.

Examples of polyglycerol esters include polyglycerol fatty acid esters obtained by esterification of one or more hydroxyl groups of polyglycerin comprising polyglycerin having a polymerization degree of 2 to 10 as a main component with fatty acid(s) having 6 to 18, preferably 12 to 18, carbon atoms, polyglycerin condensed ricinoleic acid esters and the like.

Examples of sucrose fatty acid esters include one wherein one or more hydroxyl groups of sucrose is/are esterified with fatty acid having 6 to 18, preferably 12 to 18, carbon atoms.

Examples of sorbitan fatty acid esters include sorbitans wherein one or more hydroxyl groups thereof are esterified by fatty acids having 6 to 18, preferably 12 to 18, carbon atoms, and polyoxyethylene sorbitan fatty acid esters wherein polyoxyethylene is added to hydroxyl groups of sorbitans.

Examples of lecithins include egg-yolk lecithin, purified soybean lecithin, phosphatidylcholine, phosphatidylethanolamine, phosphatidylserine, sphingomyelin, dicetyl phosphate, stearylamine, phosphatidylglycerol, phosphatidic acid, phosphatidylinositolamine, cardiolipin, ceramide phosphorylethanolamine, ceramide phosphoryl glycerol, enzymatically decomposed lecithin and a mixture thereof and the like.

HLB of the above-mentioned surfactant (E) is preferably not more than 10, more preferably not more than 7, most preferably not more than 5 because agglomeration of oil-in-water emulsified composition droplets during drying can be efficiently suppressed. Lecithins can be preferably used without any limitation on HLB, and one having an HLB of even more than 10 can also be used.
Specific example of such surfactant is a mixture of one or more kinds selected from monoglycerol monofatty acid esters such as monoglycerol monostearate, monoglycerol monooleate, monoglycerol monomyristate, monoglycerol monocaprylate, monoglycerol monolaurate, monoglycerol monobehenate, monoglycerol monoerucate and the like; monoglycerol difatty acid esters such as monoglycerol distearate, monoglycerol dioleate, monoglycerol dicaprylate, monoglycerol dilaurate and the like; fatty acid and organic acid esters of monoglycerol such as stearic acid and citric acid ester of monoglycerol, stearic acid and acetic acid ester of monoglycerol, hydrogenated coconut oil and acetic acid ester of monoglycerol, stearic acid and succinic acid ester of monoglycerol, caprylic acid and succinic acid ester of monoglycerol, stearic acid and lactic acid ester of monoglycerol, stearic acid and diacetyltartaric acid ester of monoglycerol and the like; monoglycerol fatty acid esters obtained using various fats and oils such as hydrogenated beef tallow and fatty acid esters of monoglycerol, hydrogenated canola oil and fatty acid esters of monoglycerol, hydrogenated soybean oil and fatty acid esters of monoglycerol, cottonseed oil and fatty acid esters of monoglycerol, safflower oil and fatty acid esters of monoglycerol and the like; polyglycerol esters such as polyglycerol fatty acid esters (e.g., an ester of polyglycerin having an average degree of polymerization of 2 - 10 and a fatty acid having 6 to 22 carbon atoms and the like), and polyglycerin condensed ricinoleate (e.g., an ester of polyglycerin having an average degree of polymerization of 2 - 10 and polyricinoleic acid having a condensation degree of 2 - 4 and the like) and the like; propylene glycol fatty acid esters such as propylene glycol monostearate, propylene glycol monooleate, propylene glycol monolaurate and the like; sucrose fatty acid esters such as sucrose oleate, sucrose palmitate, sucrose stearate, sucrose laurate, sucrose myristate, sucrose behenate and the like;sorbitan fatty acid esters such as sorbitan distearate, sorbitan tristearate, sorbitan sesquioleate, sorbitan dioleate, sorbitan trioleate and the like; lecithins such as soybean lecithin, egg-yolk lecithin, enzymatically decomposed lecithin and the like. Of the above-mentioned, a mixture of one or more kinds selected from glycerol fatty acid esters, polyglycerol fatty acid esters, polyglycerin condensed ricinoleates and lecithins is preferable, a mixture of one or more kinds selected from monoglycerol monofatty acid esters, monoglycerol difatty acid esters, fatty acid and organic acid esters of monoglycerol (particularly fatty acid and acetic acid esters of monoglycerol, hydrogenated coconut oil and acetic acid ester of monoglycerol), polyglycerol fatty acid esters (particularly an ester of polyglycerin having an average degree of polymerization of 2 - 10 and a fatty acid having 6 to 22 carbon atoms) and polyglycerin condensed ricinoleates (particularly an ester of polyglycerin having an average degree of polymerization of 2 - 10 and polyricinoleic acid having a condensation degree of 2 - 4) is more preferable, and fatty acid and organic acid esters of monoglycerol (particularly fatty acid and acetic acid esters of monoglycerol, hydrogenated coconut oil and acetic acid ester of monoglycerol, specifically 50% acetylated product of monoglycerol monostearate, completely acetylated product of hydrogenated coconut oil monoglyceride), polyglycerol fatty acid ester such as tetraglycerol pentaoleate, and lecithins are still more preferable.

In the production method of the present invention, the time necessary for removing water from oil-in-water emulsified composition droplets is free of any particular limitation. It is preferably within the range of 5 sec - 24 hr, more preferably 1 min - 12 hr, most preferably 5 min - 6 hr. The time necessary for removing water of less than 5 sec is not preferable because violent bubbling occurs due to instantaneous evaporation of water from oil component (B). On the other hand, the time necessary for removing water of longer than 24 hr is not preferable because producibility is degraded.

Even if water is not completely removed, removal of water in the production method of the present invention is sufficient as long as drying of oil-in-water emulsified composition droplets proceeds and recovery as particles is possible. The residual water content is generally preferably not more than 30 wt%, more preferably not more than 10 wt%, most preferably not more than 5 wt%, of the weight of recovered particles.

In the above-mentioned production method, the method of recovering the particulate composition after removal of water is not particularly limited. It is most convenient and preferable to remove oil component (B) by solid-liquid separation, wash the obtained particulate composition with an organic solvent etc. to wash away most part of oil component (B), evaporate the organic solvent and recover the composition as a powder.

Examples of the organic solvent used for washing oil component (B) include, but are not limited to, ethanol, methanol, isopropanol, acetone, hexane, ethyl acetate, tetrahydrofuran and the like. The above-mentioned organic solvent can be dried by, but is not limited to, vacuum drying, drying by heating, air drying and the like. The particulate composition after recovery may be subjected to a classification operation to have a desirable particle size of a given product.

The particulate composition comprising a bioactive substance, obtained in the present invention, can be handled, for example, packaged and crated, using glass bottles, plastic bottles, plastic bags, aluminum laminate bags and the like. In the above-described handling, for example, packaging and crating, glass, high-density polyethylene, middle-density polyethylene, low-density polyethylene, polyethylene terephthalate, polyvinyl alcohol, polypropylene, polyvinyl chloride, polyvinylidene chloride and the like, for example, can be used; materials prepared by laminating metal films such as of aluminum on the above-described polyethylene, polyethylene terephthalate and the like can also be suitably used. If a material relatively low in gas barrier quality and moisture resistance, such as polyethylene, is used, it is preferable that double or higher packaging and crating be performed with a material excellent in gas barrier quality and moisture resistance, such as aluminum laminate, for the outer bag. Using the above-described materials, PTP packaging, three-side sealed packaging, four-side sealed packaging, pillow packaging, strip packaging, aluminum-molded packaging, stick packaging and the like can be performed. After packaging and crating, the particulate composition can be transported and stored in steel drums, resin drums, fiber drums, corrugated cardboards and the like as required. Of course, an anti-moisture agent such as silica gel, calcium chloride or synthetic zeolite may be enclosed.

The particulate composition comprising a bioactive substance, obtained by the present invention, can be processed into or used as a pharmaceutical agent, food, cosmetic and the like in the form of a preparation such as tablet, pill, capsule (hard capsule, soft capsule, microcapsule and the like), chewable tablet, powder preparation, granule, syrup, drinkable preparation and the like, and the like That is, the preparation in this context does not refer solely to a pharmaceutical agent but also encompasses the aforementioned form belonging to food and cosmetics. Specifically, foods, foods with nutrient function claims, foods for specified health uses, nutritional supplements, nutritional products, animal drugs, beverages, feeds, pet food, cosmetics, pharmaceuticals, therapeutic drugs, prophylactic drugs and the like can be used. In making such preparations, excipients, disintegrants, lubricants, binders, antiflocculants, absorption promoters, solubilizers, stabilizers and the like can be used; in addition, if the particulate composition is prepared as capsules, fats and oils, surfactants and the like can be used concurrently.
Another embodiment of the present invention is an emulsified aqueous solution obtained by dissolving the particulate composition of the present invention in water. The median diameter of the emulsified particles in the emulsified aqueous solution obtained by dissolving the particulate composition of the present invention in water is almost the same as the domain average particle size of oil component (A) in the particulate composition. The median diameter affording a stable emulsified aqueous solution by dissolving the particulate composition of the present invention is normally not more than 2 µm, preferably not more than 1.5 µm. The emulsified aqueous solution obtained is applicable to health drinks, syrups, milk products and the like.

### Examples

The present invention is explained in more detail in the following by referring to Examples, which are not to be construed as limitative.

### (sphericity)

The sphericity of the obtained particulate composition was determined by analyzing, using an image analysis software (WinROOF Ver.3.30), the images obtained by observation of the recovered particles with an electron microscope and from a diameter ratio of the diameter of a circle having the same area and a smallest circumscribing circle. For the analysis, 20 samples were analyzed and the average value was determined.

### (volume average particle size)

The volume average particle size of the obtained particulate composition was measured by a laser diffraction scattering type particle size distribution measurement apparatus (Microtruck MT3000II manufactured by NIKKISO CO., LTD.) using an ethanol solvent.

### (domain average particle size)

The obtained particulate composition (50 mg) was added to distilled water (100 ml) and the mixture was completely dissolved by sonication. The emulsified particle diameter (median diameter) of the obtained aqueous solution was measured with a Scattering, Particle Size Distribution Analyzers (LB550; manufactured by HORIBA, Ltd.). The obtained data was used as the domain average particle size of the oil component (A) comprising a bioactive substance.

### Example 1

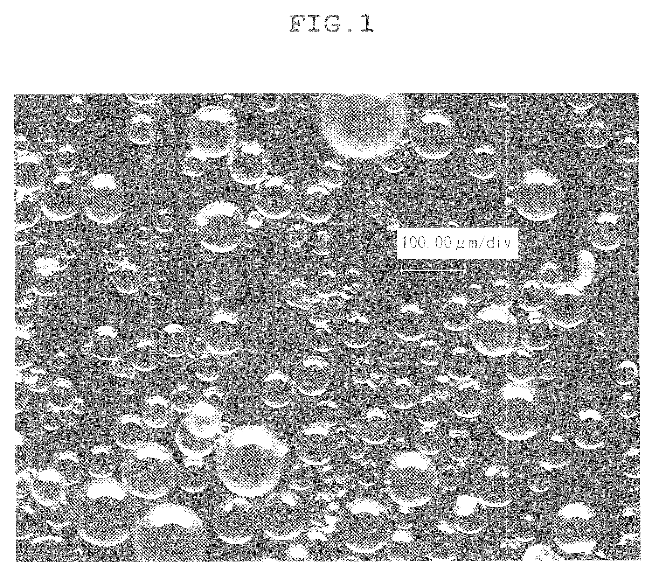

6 g of gum arabic (manufactured by Colloïdes Naturels International; EFICACIA-M) was dissolved in 24 g of distilled water at 60°C to yield a water-soluble excipient aqueous solution. Separately, 4 g of oxidized coenzyme Q10 (manufactured by Kaneka Corporation; Kaneka Coenzyme Q10) was dissolved in an oil component (A) at 60°C; this solution was added to the 60°C water-soluble excipient aqueous solution; the mixture was emulsified using POLYTRON (manufactured by KINEMATICA) at 10000 rpm for 6 minutes to yield an oil-in-water emulsified composition. The emulsified particle diameter of the oxidized coenzyme Q10 in the oil-in-water emulsified composition was about 1 µm. While stirring, 34 g of the oil-in-water emulsified composition obtained here was added to an oil component (B) consisting of 60 g of MCT (manufactured by Riken Vitamin Co., Ltd. Acter M-2) and 12 g of a surfactant (tetraglycerin pentaoleic acid ester: SY-Glyster PO-3S manufactured by Sakamoto Yakuhin Kogyo Co., Ltd., HLB3.0), previously heated to 100°C, to yield an oil-in-water-in-oil (O/W/O) emulsion. Furthermore, the emulsion was adjusted to a temperature of 105°C, and stirring was continued for 30 minutes to remove the water and form particles. Subsequently, the oil component (B) was filtered off by solid-liquid separation according to a conventional method, and the oil component (B) adhering to the particles was washed down with about 100 g of ethanol, after which the particles were dried at 40°C to yield a particulate composition comprising oxidized coenzyme Q10. The average particle size of the particulate composition obtained was 264 µm, the sphericity was 0.98, and the domain average particle size of oxidized coenzyme Q10 was 1056 nm. Morphological examination of the resulting particulate composition using a digital microscope (manufactured by Keyence Corporation; VHX-200) revealed a very beautiful sphere, as shown in Figure 1.

### <Evaluation of tabletability>

9.8 g of cellulose (manufactured by Nihon Shokuhin Kako Co., Ltd.) and 0.2 g of magnesium stearate (manufactured by Nacalai Tesque, Inc.) were added to 10 g of the particulate composition obtained, which comprised oxidized coenzyme Q10, they were blended for 1 minute, and tablets (oxidized coenzyme Q10 content 60 mg) weighing 300 mg per tablet were produced by an ordinary process for tablets. The appearance of the tablets obtained was beautiful with no tableting damage, and there were no troubles such as sticking (drug adherence to the pounder surface) during manufacturing.

### Example 2

In the same manner as Example 1, except that the amounts of gum arabic and oxidized coenzyme Q10 used were changed to 4 g and 6 g, respectively, a particulate composition comprising oxidized coenzyme Q10 was obtained. The average particle size of the composition obtained was 662 µm, the sphericity was 0.94 and the domain average particle size of oxidized coenzyme Q10 was 1845 nm.

### Comparative Example 1

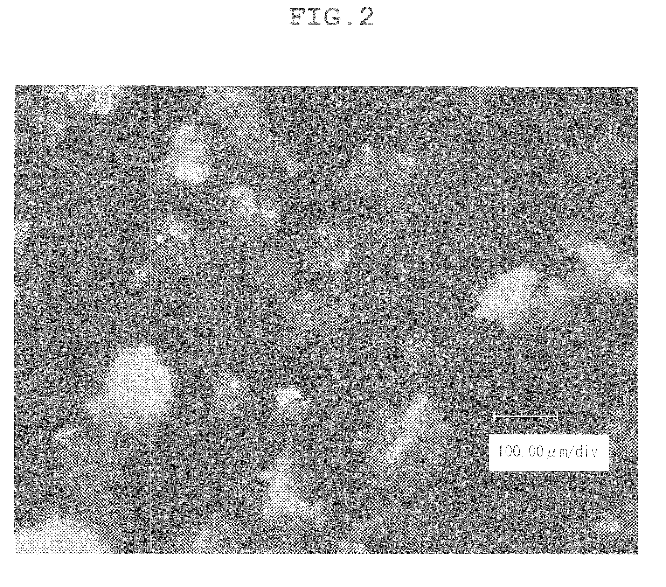

6 g of gum arabic (manufactured by Colloides Naturels International; EFICACIA-M) was dissolved in 24 g of distilled water at 60°C to yield a water-soluble excipient aqueous solution. Separately, 4 g of oxidized coenzyme Q10 (manufactured by Kaneka Corporation; Kaneka Coenzyme Q10) was dissolved in an oil component (A) at 60°C; this solution was added to the 60°C water-soluble excipient aqueous solution; the mixture was emulsified using POLYTRON (manufactured by KINEMATICA) at 10000 rpm for 6 minutes to yield an oil-in-water emulsified composition. Subsequently, spray drying was performed using a spray dryer manufactured by Nihon BUCHI K.K. (B-290) to yield a powder of oxidized coenzyme Q10. The sphericity of the powder obtained was 0.87. Morphological examination of the powder using a digital microscope (manufactured by Keyence Corporation; VHX-200) revealed a polygonal or oblong shape, as shown in Figure 2.

### Example 3: Solubility test

A particulate composition comprising oxidized coenzyme Q10 prepared by the method of Example 1 and a powder of oxidized coenzyme Q10 prepared by the spray drying method of Comparative Example 1 (these powders had the same composition of 40% oxidized coenzyme Q10 and 60% gum arabic), each 100 mg, were added to 100 ml of distilled water being warmed at 60°C, after which the mixed liquid was shaken with a mechanical shaker (120 strokes/minute, amplitude about 3 cm) for 3 minutes. After stirring, the solution was allowed to stand at room temperature in the dark for 1 day, and then examined. After further stirring for 20 minutes, the emulsified particle diameter of the solution was measured using a dynamic light scattering particle size distribution analyzer.

As a result, in the solution of the powder of oxidized coenzyme Q10 prepared by the spray drying method, a yellow powder layer of oxidized coenzyme Q10 appeared on the liquid surface, with a precipitate observed on the bottom, after standing at room temperature in the dark for 1 day. In the solution of the Q10 powder obtained by the process of Example 1, even after standing for 1 day, the powder was in uniform solution (the median diameter was 1200 nm), with no precipitate observed.

These results showed that the particulate composition obtained by the method of production of the present invention is better than the counterpart obtained by the spray drying method in terms of water solubility.

### Example 4: Evaluation of crystallinity

Using the particulate composition comprising oxidized coenzyme Q10 obtained in Example 1 and the oxidized coenzyme Q10 powder obtained in Comparative Example 1, thermal analysis using a differential scanning calorimeter (manufactured by SII; EXSTAR6000 DSC6220) was performed.

### Analytical conditions: 20°C→80°C (5°C/min)→50°C (-5°C/min)

As a result, under the heating conditions from 20°C to 80°C, the oxidized coenzyme Q10 powder of Example 1 exhibited no dissolution peak of oxidized coenzyme Q10, and during cooling from 80°C to -50°C, no crystallization peak of oxidized coenzyme Q10 existed, confirming that oxidized coenzyme Q10 was powdered in a non-crystalline state. The oxidized coenzyme Q10 powder of Comparative Example 1 exhibited a dissolution peak of oxidized coenzyme Q10 at about 50°C under the heating conditions from 20°C to 80°C, and during cooling, a crystallization peak was found; therefore, it is seen that the oxidized coenzyme Q10 was in a crystallized state.

### Example 5

5 g of gum arabic (manufactured by Colloïdes Naturels International; EFICACIA-M) was dissolved in 24 g of distilled water at 60°C to yield a water-soluble excipient aqueous solution. Separately, 5 g of oxidized coenzyme Q10 (manufactured by Kaneka Corporation; Kaneka Coenzyme Q10) was uniformly dissolved in an oil component (A) at 60°C, and the mixture was added to the 60°C water-soluble excipient aqueous solution, and the solution was emulsified using POLYTRON (manufactured by KINEMATICA) at 10000 rpm for 6 minutes to yield an oil-in-water emulsified composition. The emulsified particle diameter of the oxidized coenzyme Q10 in the oil-in-water emulsified composition was about 1 µm. While stirring, 34g of the oil-in-water emulsified composition obtained here was added to an oil component (B) consisting of 70 g of MCT (manufactured by Riken Vitamin Co., Ltd.; Acter M-2) and 2 g of a surfactant (paste lecithin: manufactured by ADM Far East K.K.; YelkinTS), previously heated to 100°C, to yield an oil-in-water-in-oil emulsion. Furthermore, the emulsion was adjusted to a temperature of 105°C, and stirring was continued for 30 minutes to remove the water and form particles. Subsequently, the oil component (B) was filtered off by solid-liquid separation according to a conventional method, and the oil component (B) adhering to the particles was washed with about 100 g of ethanol, after which the particles were dried at 40°C to yield a particulate composition comprising oxidized coenzyme Q10. The average particle size of the composition obtained was 192 µm, the sphericity was 0.97 and the domain average particle size of oxidized coenzyme Q10 was 1215 nm.

### Example 6

4 g of gum arabic (manufactured by Colloïdes Naturels International; EFICACIA-M) was dissolved in 24 g of distilled water at 60°C to yield a water-soluble excipient aqueous solution. Separately, 6 g of oxidized coenzyme Q10 (manufactured by Kaneka Corporation; Kaneka Coenzyme Q10) was uniformly dissolved in an oil component (A) at 60°C; this blend was added to the 60°C water-soluble excipient aqueous solution; the mixture was emulsified using POLYTRON (manufactured by KINEMATICA) at 10000 rpm for 6 minutes, after which it was subjected to 10 cycles of treatment at 100 MPa using a high-pressure mechanical emulsifier (manufactured by Yoshida Kikai Co., Ltd.; Nanomizer II) to yield an oil-in-water emulsified composition. The emulsified particle diameter of the oxidized coenzyme Q10 in the oil-in-water emulsified composition was about 0.8 µm. While stirring, 34 g of the oil-in-water emulsified composition obtained here was added to an oil component (B) consisting of 70 g of MCT (manufactured by Riken Vitamin Co., Ltd.; Acter M-2) and 2 g of a surfactant (paste lecithin: manufactured by ADM Far East K.K.; YelkinTS), previously heated to 80°C, after which stirring was continued under reduced pressure for 30 minutes to remove the water and form particles. Subsequently, the oil component (B) was filtered off by solid-liquid separation according to a conventional method, and the oil component (B) adhering to the particles was washed with about 100 g of ethanol, after which the particles were dried at 40°C to yield a particulate composition comprising oxidized coenzyme Q10. The average particle size of the composition obtained was 41 µm, the sphericity was 0.94, and the domain average particle size of oxidized coenzyme Q10 was 624 nm.

### Example 7

A particulate composition comprising vitamin E was prepared in the same manner as Example 5 except that 36 g of distilled water, 9 g of gum arabic (manufactured by Colloïdes Naturels International; EFICACIA-M), and 1 g of vitamin E (manufactured by Riken Vitamin Co., Ltd.; E Oil 800) instead of oxidized coenzyme Q10, were used. The average particle size of the composition obtained was 198 µm, and the sphericity was 0.95.

### Example 8

A particulate composition comprising purified fish oil was prepared in the same manner as in Example 5 except that 36 g of distilled water, 9 g of gum arabic (manufactured by Colloïdes Naturels International; EFICACIA-M), and 1 g of purified fish oil (manufactured by Kaneka Corporation) containing 6.5 wt% of docosahexaenoic acid in place of oil component (A), in which oxidized coenzyme Q10 was dissolved, were used. The average particle size of the composition obtained was 203 µm, and the sphericity was 0.93.

### Example 9

A particulate composition comprising a licorice polyphenol-containing fat and oil was obtained in the same manner as Example 5 except that 36 g of distilled water, 9 g of gum arabic (manufactured by Colloïdes Naturels International; EFICACIA-M), and 1 g of a licorice polyphenol-containing fat and oil (manufactured by Kaneka Corporation; Kaneka Glavonoid Rich Oil; licorice hydrophobic extract 10 wt% + MCT 90 wt%) in place of oil component (A) in which oxidized coenzyme Q10 was dissolved, were used. The average particle size of the composition obtained was 126 µm, and the sphericity was 0.94.

### Example 10

A particulate composition comprising oxidized coenzyme Q10 was prepared in the same manner as Example 5 except that the amounts of gum arabic and oxidized coenzyme Q10 used were changed to 6 g and 4 g, respectively, and that 0.1 g of a caramel pigment (manufactured by Ikeda Toka Industries Co., Ltd.; LF-141) was added to the water-soluble excipient aqueous solution. The average particle size of the composition obtained was 312 µm.

### Example 11

6 g of gum arabic (manufactured by Colloïdes Naturels International; EFICACIA-M) was dissolved in 24 g of distilled water at 60°C to yield a water-soluble excipient aqueous solution. Separately, 4 g of oxidized coenzyme Q10 (manufactured by Kaneka Corporation; Kaneka Coenzyme Q10) was uniformly dissolved at 60°C in an oil component (A); this blend was added to the 60°C water-soluble excipient aqueous solution; the mixture was emulsified using POLYTRON (manufactured by KINEMATICA) at 10000 rpm for 6 minutes to yield an oil-in-water emulsified composition. The emulsified particle diameter of the oxidized coenzyme Q10 in the oil-in-water emulsified composition was about 1 µm. While stirring, 34 g of the oil-in-water emulsified composition obtained here was added to an oil component (B) consisting of 70 g of MCT (manufactured by Riken Vitamin; Acter M-2) and 2 g of a surfactant (paste lecithin: manufactured by ADM Far East K.K.; YelkinTS), previously heated to 60°C, after which stirring was continued under reduced pressure for 60 minutes to remove the water and form particles. Subsequently, the oil component (B) was filtered off by solid-liquid separation according to a conventional method, and the oil component (B) adhering to the particles was washed with about 100 g of ethanol, after which the particles were dried at 40°C to yield a particulate composition comprising oxidized coenzyme Q10.

### Example 12

6 g of gum arabic (manufactured by Colloïdes Naturels International; EFICACIA-M) was dissolved in 24 g of distilled water at 60°C to yield a water-soluble excipient aqueous solution. Separately, 4 g of oxidized coenzyme Q10 (manufactured by Kaneka Corporation; Kaneka Coenzyme Q10) was dissolved in an oil component (A) at 60°C; the blend was added to the 60°C water-soluble excipient aqueous solution; the mixture was emulsified using POLYTRON (manufactured by KINEMATICA) at 10000 rpm for 6 minutes to yield an oil-in-water emulsified composition. While stirring, 34 g of the oil-in-water emulsified composition obtained here was added to an oil component (B) consisting of 68 g of a surfactant (glycerol monofatty acid ester: manufactured by Riken Vitamin Co., Ltd. Poem OL-200V, HLB 3.0), previously heated to 100°C, to yield an oil-in-water-in-oil emulsion. Furthermore, the emulsion was adjusted to a temperature of 105°C, and stirring was continued for 30 minutes to remove the water and form particles. Subsequently, the oil component (B) was filtered off by solid-liquid separation according to a conventional method, and the oil component (B) adhering to the particles was washed with about 100 g of ethanol, after which the particles were dried at 40°C to yield a particulate composition comprising oxidized coenzyme Q10. The average particle size of the composition obtained was 219 µm, and the sphericity was 0.97.

### Example 13

6 g of gum arabic (manufactured by Colloïdes Naturels International; EFICACIA-M) was dissolved in 24 g of distilled water at 60°C to yield a water-soluble excipient aqueous solution. Separately, 4 g of oxidized coenzyme Q10 (manufactured by Kaneka Corporation; Kaneka Coenzyme Q10) was dissolved in an oil component (A) at 60°C; this solution was added to the 60°C water-soluble excipient aqueous solution; the mixture was emulsified using POLYTRON (manufactured by KINEMATICA) at 10000 rpm for 6 minutes to yield an oil-in-water emulsified composition. While stirring, 34 g of the oil-in-water emulsified composition obtained here was added to an oil component (B) consisting of 66 g of MCT (manufactured by Riken Vitamin Co., Ltd. Acter M-2) and 2 g of a surfactant (lysolecithin: manufactured by Taiyo Kagaku Co., Ltd. Sunlecithin A, HLB 12.0), previously heated to 100°C, to yield an oil-in-water-in-oil emulsion. Furthermore, the emulsion was adjusted to a temperature of 105°C, and stirring was continued for 30 minutes to remove the water and form particles. Subsequently, the oil component (B) was filtered off by solid-liquid separation according to a conventional method, and the oil component (B) adhering to the particles was washed with about 100 g of ethanol, after which the particles were dried at 40°C to yield a particulate composition comprising oxidized coenzyme Q10. The average particle size of the composition obtained was 126 µm, and the sphericity was 0.97.

### Example 14

6 g of gum arabic (manufactured by Colloïdes Naturels International; EFICACIA-M) was dissolved in 24 g of distilled water at 60°C to yield a water-soluble excipient aqueous solution. Separately, 4 g of oxidized coenzyme Q10 (manufactured by Kaneka Corporation; Kaneka Coenzyme Q10) was dissolved at 60°C in an oil component (A); this solution was added to the 60°C water-soluble excipient aqueous solution; the mixture was emulsified using POLYTRON (manufactured by KINEMATICA) at 10000 rpm for 6 minutes to yield an oil-in-water emulsified composition. While stirring, 34 g of the oil-in-water emulsified composition obtained here was added to an oil component (B) consisting of 60 g of a food oil (rapeseed refined oil: manufactured by Kaneka Corporation) and 8 g of a surfactant (tetraglycerin pentaoleic acid ester: SY-Glyster PO-3S manufactured by Sakamoto Yakuhin Kogyo Co., Ltd., HLB 3.0), previously heated to 100°C, to yield an oil-in-water-in-oil emulsion. Furthermore, the emulsion was adjusted to a temperature of 105°C, and stirring was continued for 30 minutes to remove the water and form particles. Subsequently, the oil component (B) was filtered off by solid-liquid separation according to a conventional method, and the oil component (B) adhering to the particles was washed with about 100 g of ethanol, after which the particles were dried at 40°C to yield a particulate composition comprising oxidized coenzyme Q10. The average particle size of the composition obtained was 156 µm and the domain average particle size of oxidized coenzyme Q10 was 1014 nm.

### Example 15

A particulate composition comprising oxidized coenzyme Q10 was prepared in the same manner as Example 1 except that 24 g of distilled water, 4 g of gum arabic (manufactured by Colloïdes Naturels International; EFICACIA-M), and 2 g of sucrose (manufactured by Wako Pure Chemical Industries, Ltd.) were used as the water-soluble excipient aqueous solution. The average particle size of the composition obtained was 194 µm.

### Example 16

A particulate composition comprising oxidized coenzyme Q10 was obtained in the same manner as Example 1 except that 36 g of distilled water, 5 g of ghatti gum (manufactured by Colloides Naturels International) and 5g of oxidized coenzyme Q10 were used as the water-soluble excipient aqueous solution. The average particle size of the composition obtained was 160 µm.

### Example 17

A particulate composition (100 mg) containing oxidized coenzyme Q10, which had been prepared by the method of Example 1, was added to distilled water (100 ml) and completely dissolved and emulsified by sonication. The obtained emulsified aqueous solution was stood for one day. As a result, the emulsified solution remained uniform and free of precipitation and creaming. The median diameter of the emulsified particle (oxidized coenzyme Q10 droplet) in the obtained emulsified aqueous solution was about 1 µm.

While some of the embodiments of the present invention have been described in detail in the above, those of ordinary skill in the art can enter various modifications and changes to the particular embodiments shown without substantially departing from the novel teaching and advantages of the present invention. Such modifications and changes are encompassed in the spirit and scope of the present invention as set forth in the appended claims.

This application is based on application Nos. 2006-295426 and US provisional application No. 60/857111, the contents of which are incorporated hereinto by reference.

## Claims

1. A particulate composition wherein an oil component (A) comprising a poorly water-soluble bioactive substance is poly-dispersed while forming a domain in a matrix comprising a water-soluble excipient comprising a water-soluble polymer as a main component, and wherein the sphericity is not less than 0.9.

2. The particulate composition of claim 1, wherein the poorly water-soluble bioactive substance is at least one kind selected from the group consisting of oxidized coenzyme Q10, an unsaturated fatty acid, an oil-soluble vitamin, a plant hydrophobic extract ingredient and a carotenoid.

3. The particulate composition of claim 1 or 2, wherein the oil component (A) is poly-dispersed while forming not less than five domains.

4. The particulate composition of any one of claims 1 to 3, wherein the water-soluble polymer is at least one kind selected from the group consisting of gum arabic, ghatti gum, agar, starch, pectin, carrageenan, casein, dried albumen, curdlan, alginic acids, soybean polysaccharide, pullulan, celluloses, xanthan gum, carmellose salt and polyvinylpyrrolidone.

5. The particulate composition of claim 2, wherein the unsaturated fatty acid is at least one kind selected from the group consisting of oleic acid, linoleic acid, conjugated linoleic acid, linolenic acid, arachidonic acid, docosahexaenoic acid, docosapentaenoic acid and eicosapentaenoic acid.

6. The particulate composition of claim 2, wherein the oil-soluble vitamin is at least one kind selected from the group consisting of vitamin A, vitamin D, vitamin E, vitamin K, and derivatives thereof.

7. The particulate composition of claim 2, wherein the plant hydrophobic extract ingredient is obtained from at least one kind of plants or plant-processed products selected from the group consisting of licorice, turmeric, perilla, clove, cinnamon, ginger, lemongrass, peppermint, dokudami (Houttuyania cordata), coix seed, rice bran, cornflower, fennel, boxthorn, zanthoxylum, nasturtium, dioscorea, sanryo, kinkaran (Tinospora capillipes), amachazuru (Gynostema pentaphyllum), thuja, hakutouou (Pulsatilla chinensis), parsley, onion, nutmeg, wild-rice, gluten feed, tobiko (konnyaku byproduct powder), paprika, horseradish, lemon, capsicum, sesame, spearmint and leaf mustard.

8. The particulate composition of any one of claims 1 to 6, wherein the oil component (A) comprises 1 to 100 wt% of a bioactive substance, 0 to 99 wt% of a fat and oil, and 0 to 99 wt% of a surfactant (D).

9. The particulate composition of claim 8, wherein the surfactant (D) is at least one kind selected from the group consisting of glycerol fatty acid ester, polyglycerin ester, sucrose fatty acid ester, sorbitan fatty acid ester, and propylene glycol fatty acid ester, each having an HLB of not more than 10, and lecithins.

10. The particulate composition of any one of claims 1 to 9, wherein the content of the bioactive substance in the particulate composition is 1 - 70 wt%.

11. The particulate composition of any one of claims 1 to 10, wherein the volume average particle size is 1 - 1000 µm.

12. The particulate composition of any one of claims 1 to 11, wherein the average particle size of the domain(s) is 0.01 to 50 µm.

13. The particulate composition of any one of claims 1 to 12, wherein a light-fast ingredient is contained in the composition.

14. The particulate composition of claim 13, wherein the light-fast ingredient is at least one kind selected from the group consisting of titanium oxide, food colors, red iron oxide pigment, safflower pigment, annatto pigment, caramel pigment, gardenia pigment, tar pigment and chlorophyll.

15. An emulsified aqueous solution prepared by dissolving the particulate composition of any one of claims 1 to 14 in water, wherein a median diameter of the emulsified particles in emulsified aqueous solution is not more than 2 µm.

16. A method of producing a particulate composition comprising a bioactive substance, comprising suspending an oil-in-water emulsified composition prepared from an oil component (A) comprising a poorly water-soluble bioactive substance and an aqueous solution comprising a water-soluble excipient comprising a water-soluble polymer as a main component in an oil component (B), and thereafter removing the water in the emulsified composition in the oil component (B).

17. The method of claim 16, wherein the oil component (B) comprises 0 - 99.99 wt% of fat and oil and 0.01 - 100 wt% of surfactant (E).

18. The method of claim 16, wherein the surfactant (E) is at least one kind selected from the group consisting of glycerol fatty acid ester, polyglycerin ester, sucrose fatty acid ester and sorbitan fatty acid ester, each having an HLB of not more than 10, and lecithins.

19. The method of any one of claims 16 to 18, wherein the obtained particulate composition has a sphericity of not less than 0.9.

20. The method of any one of claims 16 to 19, wherein the water-soluble polymer is at least one kind selected from the group consisting of gum arabic, ghatti gum, agar, starch, pectin, carrageenan, casein, dried albumen, curdlan, alginic acids, soybean polysaccharide, pullulan, celluloses, xanthan gum, carmellose salt and polyvinylpyrrolidone.

21. The method of production of any one of claims 16 to 20, wherein the oil component (A) comprises 1 to 100 wt% of a bioactive substance, 0 to 99 wt% of a fat and oil, and 0 to 99 wt% of a surfactant (D).

22. The method of production of any one of claims 16 to 21, wherein the poorly water-soluble bioactive substance is at least one kind selected from the group consisting of oxidized coenzyme Q10, an unsaturated fatty acid, an oil-soluble vitamin, a plant hydrophobic extract ingredient and a carotenoid.

23. The method of claim 21, wherein the surfactant (D) is at least one kind selected from the group consisting of glycerol fatty acid ester, polyglycerin ester, sucrose fatty acid ester, sorbitan fatty acid ester and propylene glycol fatty acid ester, each having an HLB of not more than 10, and lecithins.
